# EUROPEAN PATENT APPLICATION

(11) **EP 1 649 836 A2**
(43) Date of publication of application: **26.04.2006**
(21) Application number: 05109632.9
(22) Date of filing: 17.10.2005
(51) Int. Cl.: A61F 2/40

(54) **Inverse prosthesis for the shoulder joint**

(30) Priority: 20.10.2004 IT UD20040195
(71) Applicant: LIMA Lto SpA, 33030 Villanova di San Daniele del Friuli (IT)
(72) Inventor: Dalla Pria, Paolo, 33100, UDINE (IT)
(74) Representative: Petraz, Davide Luigi

(57) **Abstract**

Inverse prosthesis (10) for the articulation of a humerus (12) in a scapula (11) of a shoulder having a glenoid cavity (15). The inverse prosthesis (10) comprises a first articulation element (20), at least partly concave, associated with the top of the humerus (11), and a second articulation element (19), partly convex, associated with the glenoid cavity (15). The inverse prosthesis (10) also comprises a concave insert (14) interposed between the first (20) and the second (19) articulation element, substantially hemispherical in shape, at least partly housed in the first articulation element (20) and free to move with respect thereto, and in which the second articulation element (19) articulates.

## Description

### FIELD OF THE INVENTION

The present invention concerns a prosthesis of the shoulder for the articulation of the head of a humerus with respect to the relative scapula. To be more exact, the prosthesis according to the present invention is configured as an inverse prosthesis, wherein a concave artificial seating is associated with the top of the humerus and articulates, by means of an insert housed inside it, with a convex-shaped head associated with the glenoid cavity.

### BACKGROUND OF THE INVENTION

Inverse prostheses of the shoulder are known, comprising a spherical element attached to the scapula which articulates in a mating cavity obtained in the upper part of the humerus. Such prostheses are called "inverse" because they reproduce in an inverse manner the gleno-humeral anatomy.

Normally, inverse prostheses are used in cases of serious muscular degeneration of the shoulder, particularly of the muscles of the rotator cuffs. This degeneration causes a prevalence of the action of the deltoid, which tends to exert a traction of the humerus upwards, consequently making the bony head of the humerus, or possibly an artificial head, move upwards, with the risk of making it knock against the protrusion which extends the spine of the spatula, called acromion.

Inverse prostheses comprise a concave-shaped humeral seating associated with an attachment rod grafted into the humerus. The humeral seating normally consists of a cup made of plastic material, for example polyethylene, which articulates directly with a spherical element attached to the glenoid cavity.

It is known that, to prevent dislocations, or sub-dislocations, of the artificial articulation grafted in, which in some cases can even lead to a surgical intervention, the plastic cup substantially surrounds the spherical element, so as to guarantee a high coverage and prevent the spherical element from coming out.

This requirement for a wide covering surface of the cup entails, however, not only a natural reduction in the angle of rotation of the humerus with respect to the scapula, but also a frequent contact and rubbing of the edge of the cup and the bony part of the scapula.

This rubbing initially determines an effect of mechanical scraping of the bony part of the scapula, and subsequently, due to the release of plastic particles, an effect of osteolysis which causes an enormous re-absorption of the bone. This re-absorption of the bone, also known under the technical name of "scapular notch", endangers the mechanical stability of the part of the prosthesis constrained to the glenoid cavity, determining a limited duration of the correct functioning of the prosthesis.

One purpose of the present invention is to achieve an inverse prosthesis for the articulation of the shoulder in which the problem of rubbing of the polyethylene part of the prosthesis against the bony part is substantially eliminated, guaranteeing in any case a great safety level against dislocations or sub-dislocations of the articulation.

Another purpose is to ensure a great travel of the humerus with respect to the scapula, substantially irrespective of the size of the cup associated with the humerus.

The Applicant has devised, tested and embodied the present invention to overcome the shortcomings of the state of the art and to obtain these and other purposes and advantages.

### SUMMARY OF THE INVENTION

The present invention is set forth and characterized in the main claim, while the dependent claims describe other characteristics of the invention or variants to the main inventive idea.

In accordance with the above purposes, an inverse prosthesis according to the present invention comprises a first articulation element, or humeral cup, at least partly concave and associated with the top of the humerus, and a second articulation element, or glenoid head, partly convex and associated with the natural cavity of the scapula.

According to the present invention, the inverse prosthesis also comprises a concave insert, interposed between the first and the second articulation element, substantially hemispherical in shape, at least partly housed in the first articulation element, free to move with respect thereto, and in which the second articulation articulates.

The concave insert, allowing the free mobility of the humeral cup with respect thereto, allows an extra travel of rotation of the humeral cup both in the limit positions on a longitudinal plane (with the arm along the side or raised therefrom), and also in those limit positions on a transverse plane (with the arm resting on the trunk or spread out with respect to it).

The present invention thus guarantees a wide and safe articulation travel of the shoulder, with no risk of dislocations or sub-dislocations, even using humeral cups with a limited surface extension. Moreover, in the limit positions of the articulation, the insert, or the cup, once in contact with the bony part of the scapula, do not rub against it, since the movement of articulation is transformed into a relative movement between cup and insert, which limits the wear of the bony part to a minimum.

This configuration allows to use an insert made of plastic polyethylene material, for example polyethylene with a high molecular weight (UHMWPE) or polyethylene with high reticulation, of which the problems are known connected to the wear of the bony part, since the situations of potential rubbing are reduced to a minimum. In fact, in the case of an intermediate insert protruding from the cup, in the limit positions it first abuts against the bony part, but thanks to its mobility with respect to the cup, a further movement of the articulation translates into a sliding of the insert towards the inside of the cup, so that no rubbing takes place.

In the case of an insert completely contained inside the cup, it is the edge of the cup which contacts the bony part first, but in this case too a further movement of the articulation simply determines a movement of the insert with respect to the cup.

According to a variant, on the bottom of the cup there are means able to impart a rotation of the insert in one or more preferential directions.

The invention, as we said, allows to use an insert made of polyethylene material, so as to be able to exploit the excellent behavior of this material in terms of low friction to sliding and reduced wear. At the same time, it is possible to use humeral cups made of metal material, since there is no contact with the glenoid head.

According to other solutions, it is possible to make the humeral cup in polyethylene material too, or the glenoid head itself, or to make all three components in ceramic material.

In one form of embodiment, the insert covers an angle of about 180°, so as to assume the shape substantially of a semispherical cap. According to constructional variants, the insert has a surface that covers angles of about 150°, 120° or 90°.

According to a variant, when it is made of polyethylene material, the humeral cup has an outer edge covered with inert material, such as metal, ceramic or other, so as to further reduce the consequences deriving from its possible contact against the bony part of the scapula.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 shows a longitudinal section of a prosthesis of the inverse type according to the invention in a first operating position;
- fig. 2 shows a longitudinal section of the prosthesis in fig. 1, in a second operating position;
- fig. 3 is an exploded view of some components of the prosthesis in fig. 1;
- figs. 4 and 5 show a transverse section of two constructional variants of a detail of the prosthesis in fig. 1;
- fig. 6 is a three-dimensional view of a variant of the prosthesis in fig. 1;
- fig. 7 shows a transverse section of the prosthesis in fig. 6.

### DETAILED DESCRIPTION OF A PREFERENTIAL EMBODIMENT

With reference to the attached drawings, the number 10 denotes generally an inverse prosthesis according to the present invention, able to be implanted in a shoulder, to allow the articulation of a humerus 12 in a relative glenoid cavity 15 of a scapula 11, only partly shown here.

To be more exact, the prosthesis 10 comprises, on the side of the scapula 11, a supporting element, not shown, which allows to attach and position a glenoid head 19 and, on the side of the humerus 12, an anchoring rod 38 which allows to attach and position, by means of a humeral support body 37, a humeral cup 20.

An intermediate insert 14, concave in shape, is housed in sliding manner in the humeral cup 20. The insert 14 substantially consists of a hemispherical cap, and the glenoid head 19 articulates in the inner surface 16 thereof.

The humeral cup 20 defines an inner articulation seating 23, of a shape correlated to an outer convex surface 17 of the insert 14.

The insert 14 is advantageously made of polyethylene plastic material and comprises, as we said, a concave inner surface 16 in which the glenoid head 19 is partly housed, and a convex outer surface 17 partly housed in the articulation seating 23 of the humeral cup 20.

The insert 14 also comprises a circular edge 27 which, in the case of an insert protruding from the cup, disposes itself, in the limit positions, in contact with the bony part of the scapula 11.

The insert 14, being mobile with respect to the humeral cup 20, allows an extra travel of the latter with respect to the glenoid head 19 in the limit positions, considerably reducing the risk of dislocations or sub-dislocations even in the case as shown here, with humeral cups 20 of very limited size.

Moreover, thanks to this relative mobility of the insert 14 and cup 20, there is no risk of rubbing of the edge 27 even when it is in contact with the bony part of the scapula 11, since a further movement of the articulation is transformed, precisely, into a relative movement between insert 14 and cup 20. Thanks to this, the risks of re-absorption of the bone due to scapular notch are reduced to a minimum.

Moreover, even in the case when the insert 14 is of a size such that it is always contained inside the humeral cup 20, with the edge of the humeral cup 20 that first contacts the bony part of the scapula 11, in any case there is no rubbing since the relative mobility between the cup 20 and the insert 14 is exploited in order to allow a further travel of the articulation.

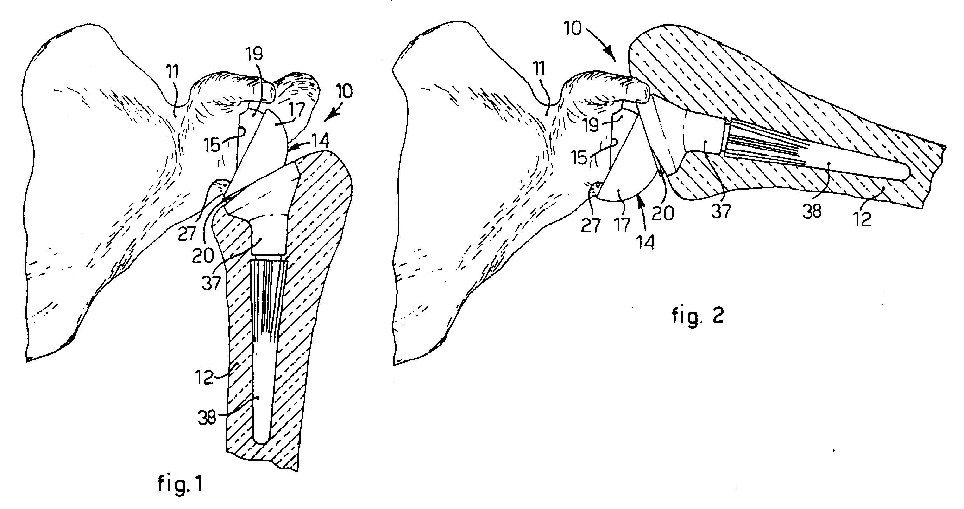

The solution shown in figs. 1 to 5 refers to the case where the insert 14 can move freely in all directions based on the stresses received following the movement of the articulation. In a variant, not shown here, the bottom of the cup 20 includes means, such as ridges, pins, guides or other similar means, to impose on the insert 14 a movement in a single preferential direction.

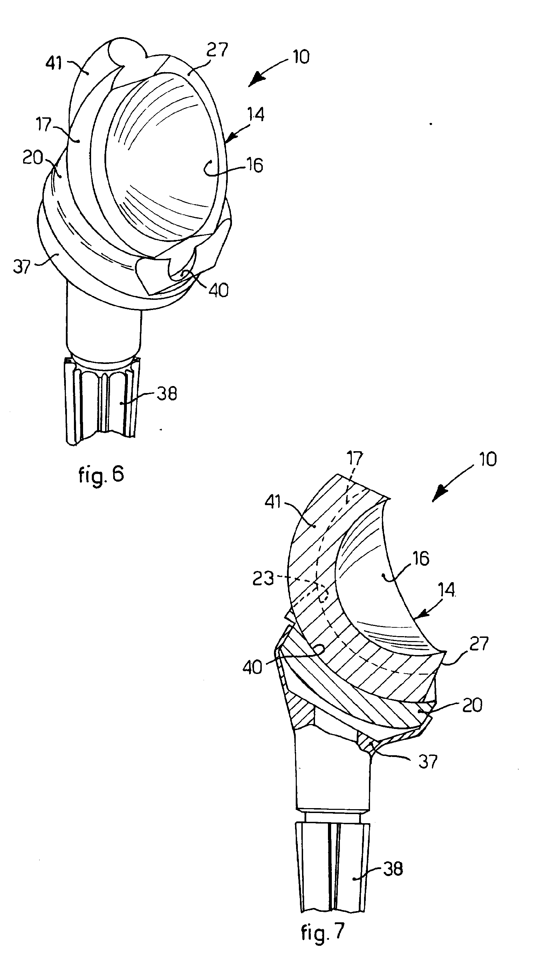

In the solution shown in figs. 6 and 7, in order to achieve said preferential movement, on the surface of the articulation seating 23 of the cup 20 a guide channel 40 is made, inside which a mating ridge 41, provided on the convex surface 17 of the insert 14, is able to slide in guided manner.

In this way, the articulation movement of the insert 14 in the cup 20 is constrained to the development of the guide channel 40.

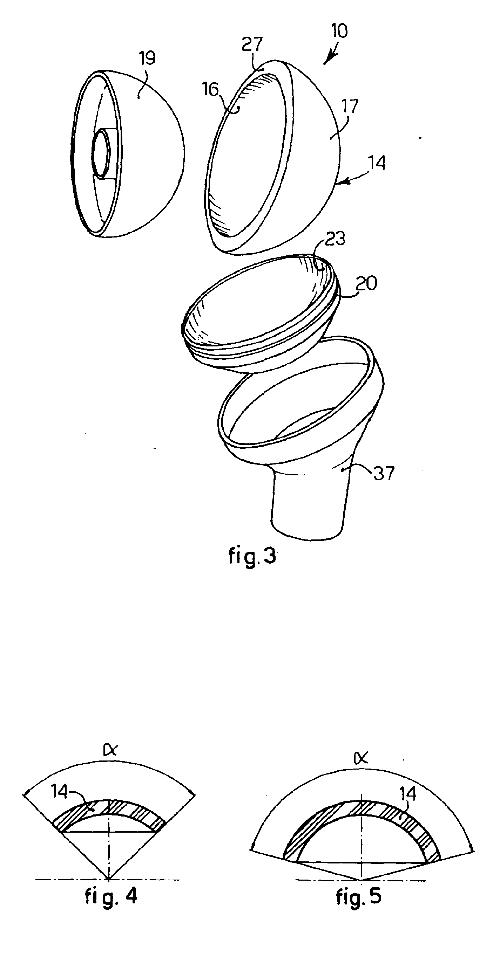

According to the variants shown schematically in figs. 4 and 5, the insert 14 has an extension such as to cover respectively an angle α of about 90°, and respectively about 150°, so as to consequently limit the possibilities of travel of the articulation.

It is clear however that modifications and/or additions of parts may be made to the inverse prosthesis 10 as described heretofore, without departing from the field and scope of the present invention.

For example, it comes within the field of the present invention to provide any combination of material for the glenoid head 19, the humeral cup 20 and the intermediate insert 14, including metal, plastic and ceramic material, although it is in any case preferable that the intermediate insert 14 is always made of polyethylene material.

It also comes within the field of the present invention to provide that on the outer edge of the humeral cup 20, when it is made of polyethylene material, a covering ring may be provided, made of anti-wear inert material, to prevent the rubbing between the humeral cup 20 and the scapula 11 from causing the start of a process of wear and degeneration of the bony part of the latter.

In another possibility, the concave surface 16 of the insert 14 has a coefficient of friction greater than the surface of the concave surface 17, or of the glenoid head 19, so as to facilitate limited movements of reciprocal drawing, which are transformed into reciprocal sliding only in the limit positions of the articulation.

It is also clear that, although the present invention has been described with reference to specific examples, a person of skill in the art shall certainly be able to achieve many other equivalent forms of inverse prosthesis for the articulation of the shoulder, having the characteristics as set forth in the claims and hence all coming within the field of protection defined thereby.

## Claims

1. Inverse prosthesis of the shoulder for the articulation of a humerus (12) in a scapula (11) of a shoulder having a glenoid cavity (15), said prosthesis comprising a first articulation element (20), at least partly concave, associated with the top of said humerus (11), and a second articulation element (19), partly convex, associated with said glenoid cavity (15), **characterized in that** it also comprises a concave insert (14) interposed between said first (20) and said second (19) articulation element, substantially hemispherical in shape, at least partly housed in said first articulation element (20) and free to move with respect thereto, and in which said second articulation element (19) articulates.

2. Prosthesis as in claim 1, **characterized in that** said insert (14) is made of plastic material.

3. Inverse prosthesis as in claim 1 or 2, **characterized in that** said insert (14) is disposed during use at least partly protruding from said first articulation element (20).

4. Inverse prosthesis as in claim 1 or 2, **characterized in that** said insert (14) is disposed during use completely inside said first articulation element (20).

5. Prosthesis as in any claim hereinbefore, **characterized in that** said insert (14) comprises a convex outer surface (17) able to be at least partly housed in a concave articulation seating (23) of said first articulation element (20), and a concave inner surface (16) in which an outer surface of said second articulation element (19) is partly housed.

6. Inverse prosthesis as in claim 5, **characterized in that** on the bottom of said articulation seating (23) there are means able to impose a movement according to at least a preferential direction on said insert (14).

7. Prosthesis as in claim 6, **characterized in that** said means able to impart a movement in at least a preferential direction to said insert (14) comprise at least a guide channel (40) made on said surface of said articulation seating (23), and a ridge (41) made on said convex surface (17) and able to slide inside said guide channel (40).

8. Prosthesis as in any claim hereinbefore, **characterized in that** said insert (14) has an extension such as to cover an angle comprised between about 30° and about 180°.

9. Prosthesis as in any claim hereinbefore, **characterized in that** said first articulation element comprises a humeral cup (20) made of metal, ceramic material or plastic material, and said second articulation element comprises a glenoid head (19) made of metal, ceramic material or plastic material.
